(19) Europäisches Patentamt
European Patent Office
Office européen des brevets

(11) Publication number: **0 029 924**
**B1**

# (12) EUROPEAN PATENT SPECIFICATION

(45) Date of publication of patent specification: **15.08.84**

(51) Int. Cl.³: **C 07 J 5/00**, A 61 K 31/57 //C07J71/00

(21) Application number: **80106720.8**

(22) Date of filing: **03.11.80**

## (54) 6-Alpha-fluoro-16-methyl-prednisolone-17,21 diesters and pharmaceutical compositions containing them.

(30) Priority: **16.11.79 IT 2735379**
**09.10.80 IT 4985380**

(43) Date of publication of application:
**10.06.81 Bulletin 81/23**

(45) Publication of the grant of the patent:
**15.08.84 Bulletin 84/33**

(84) Designated Contracting States:
**AT BE CH DE FR GB LI LU NL SE**

(56) References cited:
**DE - A - 2 649 097**
**GB - A - 924 931**
**GB - A - 933 859**
**US - A - 4 018 918**

(73) Proprietor: **STEROSYNT Ltd.**
**10, Elmdale Road**
**Bristol (GB)**

(72) Inventor: **Macdonald, Peter**
**Viale Sempione 21/35**
**Arese (Milan) (IT)**

(74) Representative: **Bianchetti, Giuseppe**
**Studio Consulenza Brevettuale Via Senato 24**
**I-20121 Milan (IT)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 029 924 B1

**Description**

The invention relates to novel 6α-fluoro-prednisolone 17,21-diesters having the formula I,

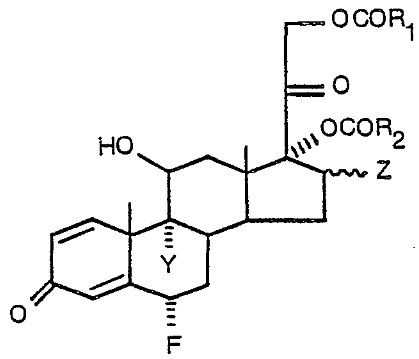

(I)

in which $R_1$ and $R_2$ are alkyl groups containing 1—6 carbon atoms or aryl groups, Y is H, Z is α-$CH_3$ or β-$CH_3$.

In U.S.—A—4018918 and GB—A—1403962 there are disclosed compounds having an halogen atom in the 9 position and they are therefore different from the compounds of formula I. The process described in GB—A—1403962 is also different referring always to hydrofluorination of a 9β,11β-epoxide and nowhere in the reference cited is found an example of either hydrobromination or debromination.

In G.B.—A—924931 and 933859, referring to halo-pregnene derivatives, a very broad general formula is claimed, but no physical characteristics and no pharmaceutical properties are given.

Further, none of the examples or claims refer to 6α-fluoro-9α-chloroprednisolone 17,21-diesters-the diesters mentioned all lack the vital 11β-hydroxy function and no pathway is indicated which would lead to such compounds (the methods mentioned for obtaining 17-esters would acetylate any 11β-hydroxy group and destroy the antiinflammatory activity).

In our Italian application No. 25149 A/79 there is described a novel and general method for the preparation of 6α-fluoro-9β,11β-epoxy-pregna-1,4-dienes having the general formula II

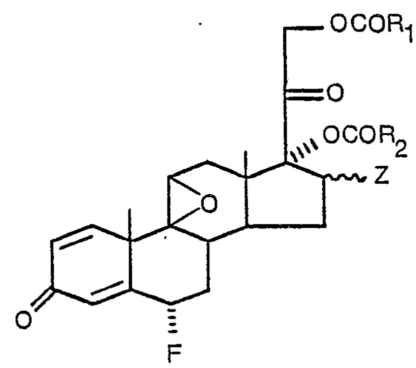

(II)

where $R_1$, $R_2$ and Z have the meanings given above, from the corresponding 3-acetoxy-9β,11β-epoxy-pregna-1,3,5-trienes of general formula III

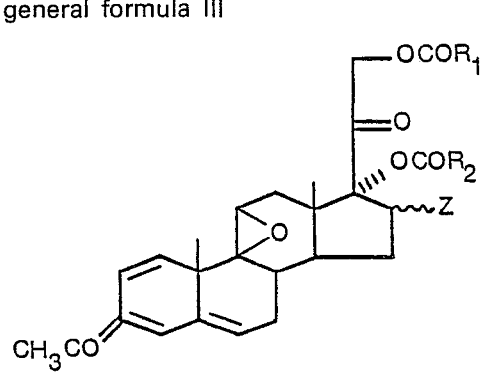

(III)

where $R_1$, $R_2$, and Z have the meanings given above.

In our Italian application 26227 A/79 there is described an alternative method for the preparation

of 6$\alpha$-fluoro-9$\beta$,11$\beta$-epoxy-pregna-1,4-dienes having the general formula II from the corresponding 1,2-dihydro-compounds having the general formula IV,

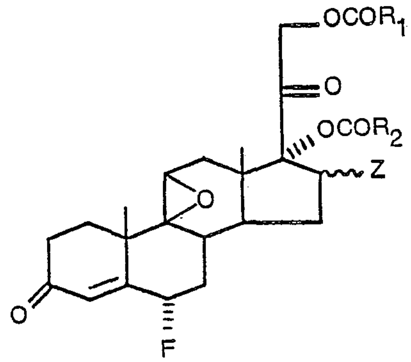

(IV)

where $R_1$, $R_2$, and Z have the meanings given above.

In the above-mentioned application there is also described the conversion of certain compounds of general formula II, into known corticoids such as diflorasone diacetate and 6$\alpha$,9$\alpha$-difluoro-prednisolone 17,21-diacetate.

It has now been found that the compounds of general formula II, where $R_1$, $R_2$, and Z have the meanings given above, may also be converted using known methods into novel 6$\alpha$-fluoro-16-methyl-prednisolone 17,21-diesters having the general formula I and that certain of these novel compounds possess exceptionally high antiinflammatory activity.

For example, reaction of the compound of formula II ($R_1$ = methyl, $R_2$ = butyl, Z = 16-$\alpha$-methyl) with hydrogen bromide gives 6$\alpha$-fluoro-9$\alpha$-bromo-6$\alpha$-methyl-prednisolone 17-valerate 21-acetate (I; $R_1$ = methyl, $R_2$ = butyl, Y = Br, Z = 16$\alpha$-methyl), which on subsequent treatment with tributyltin hydride affords 6$\alpha$-fluoro-16$\alpha$-methyl-prednisolone 17-valerate 21-acetate (I; $R_1$ = methyl, $R_2$ = butyl, Y = H).

The reaction of the compounds of formula II with hydrogen bromide is carried out under conditions usual for the bromination of epoxy steroids; the hydrogen bromide being used anhydrous, aqueous, or generated *in situ* from lithium bromide and glacial acetic acid. The preferred method of hydrobromination is to treat a solution of the epoxide in acetic acid or carbon tetrachloride with a solution of hydrogen bromide in glacial acetic acid.

The reaction of the compounds of formula I (Y = Br) with tributyltin hydride to give the corresponding compounds of formula I (Y = H) is usually carried out in anhydrous tetrahydrofuran at reflux, preferably in the presence of an initiator of free radical reactions such as 2,2′-azobisisobutyronitrile.

Although the preferred method for preparing the compounds of general formula I is from the corresponding 9$\beta$,11$\beta$-epoxide of general formula II, in certain cases other methods may be conveniently used. Thus, the novel 6$\alpha$-fluoro-16-methyl-prednisolone 17,21-diesters of general formula I may be obtained from the corresponding 6$\alpha$-fluoro-16-methyl-prednisolone by conversion to a 17,21-alkylorthoalkanoate ester by known procedures, followed by cleavage thereof with acid using known techniques to give the corresponding 17-alkanoate ester, followed by acylation in position 21 using standard procedures.

Another method for the preparation of the compounds of general formula I which may occasionally be convenient is by dehydrogenation of the corresponding 1,2-dihydro compounds having the general formula V,

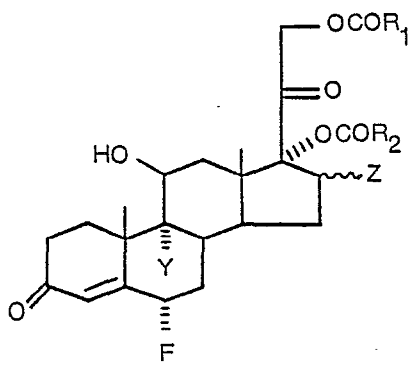

(V)

wherein $R_1$, $R_2$, and Z have the meanings given above, using either chemical or microbiological methods.

The preparation of the compounds having the general formula V from the corresponding compounds of general formula IV is described in our Italian appln. 26227 A/79. However it has been

found that the dehydrogenation of the compounds of general formula V with dichlorodicyanobenzoquinone (which is the preferred chemical agent for dehydrogenation) shows rather low yields (70—80%); while the same method of dehydrogenation, applied to the compounds of general formula IV, gives 1,4-dienes of formula II in high yields (90—95%). Thus the preferred route from compound IV to compound I is usually via compound II rather than via compound V.

Preferred compounds of formula I include:

the 21-acetate, 21-propionate, 21-butyrate, 21-isobutyrate, 21-valerate, and 21-hexanoate ester derivatives of

6α-fluoro-16α-methyl-prednisolone 17-acetate
6α-fluoro-16α-methyl-prednisolone 17-propionate
6α-fluoro-16α-methyl-prednisolone 17-butyrate
6α-fluoro-16α-methyl-prednisolone 17-valerate
6α-fluoro-16α-methyl-prednisolone 17-benzoate

the 21-acetate, 21-propionate, 21-butyrate, 21-isobutyrate, and 21-valerate ester derivatives of

6α-fluoro-16β-methyl-prednisolone 17-acetate
6α-fluoro-16β-methyl-prednisolone 17-propionate
6α-fluoro-16β-methyl-prednisolone 17-butyrate
6α-fluoro-16β-methyl-prednisolone 17-valerate
6α-fluoro-16β-methyl-prednisolone 17-benzoate

Of the foregoing compounds particularly valuable are the following ones (indicated with a reference number):

105) 6α-fluoro-16α-methyl-prednisolone 17,21-diacetate
106) 6α-fluoro-16α-methyl-prednisolone 17,21-dipropionate
107) 6α-fluoro-16α-methyl-prednisolone 17-valerate 21-acetate
108) 6α-fluoro-16α-methyl-prednisolone 17-benzoate 21-acetate
109) 6α-fluoro-16β-methyl-prednisolone 17,21-diacetate
110) 6α-fluoro-16β-methyl-prednisolone 17,21-dipropionate
111) 6α-fluoro-16β-methyl-prednisolone 17-valerate 21-acetate
112) 6α-fluoro-16β-methyl-prednisolone 17-benzoate 21-acetate.

The compounds of the present invention may be used in a method of treating an inflammatory condition in a warm-blooded animal responsive to treatment with antiinflammatory agents which comprises administering to said animal a non-toxic, antiinflammatory effective amount of 6α-fluoroprednisolone 17,21-diester of general formula I. The preferred compounds of formula I are valuable antiinflammatory agents when administered topically, or locally, since they have high antiinflammatory action as well as low glucocorticoid action on topical administration, and moreover have low glucocorticoid activity when administered systematically. The compounds thus have the desirable high antiinflammatory action on topical application with little risk of disturbance of the mineral balance or other systemic action should the compound be absorbed.

The 6α-fluoro-16-methyl-prednisolone 17,21-diesters of formula I may be applied topically or locally in any of the conventional pharmaceutical forms, including ointments, lotions, creams, sprays, powders, drops (ear drops or eye drops), suppositories, tablets, pellets, or aerosols.

Biological activity

The biological activity of the compounds of the invention was compared to that of beclomethasone dipropionate using the cotton pellet granuloma assay. Female rats (Sprague-Dawley) weighing about 135 g were used and into each animal, under ether anaesthesia, was implanted subcutaneously a cotton pellet weighing 10 mg. The cotton pellet were previously soaked with 25 ml of a solution of the test substance and with 50 ml of a 2% carrageenin suspension and left to dry. The cotton pellets contained 0.1, 1, or 10 mg of the test substance (ten animals were used for each concentration of each substance).

After seven days the animals were sacrificed and the granulomas that had formed around the cotton pellets were removed, dried at 80°C and weighed. The adrenal and thymus glands were also removed and weighed.

A similar procedure was carried out using beclomethasone dipropionate, as well as a control.

The results are summarized in Table 1, with the corresponding reference numbers.

It is seen from these results that the compounds cause up to 46.4% inhibition of granuloma formation at dosages of 0.1 mg/rat whereas beclomethasone dipropionate was inactive at this low dosage.

The more active compounds have an activity comparable with 100-fold dosages of

beclomethasone dipropionate. Notwithstanding this elevated antiinflammatory potency the compounds of the invention usually had no significant effects on the weights of the thymus and adrenal glands even at levels 100 times greater than the effective antiinflammatory dosages.

TABLE 1

| Compound | Dose (mcg/rat) | % Variation with respect to the control: cotton pellet | adrenal gland | thymus gland |
|---|---|---|---|---|
| "106" | 0.1 | +0.9 | +0.4 | −2.3 |
| | 1 | −2.2 | −1.6 | −2.6 |
| | 10 | −5.0 | 0 | −3.8 |
| "107" | 0.1 | −38.8 | −3.0 | −6.1 |
| | 1 | −52.5 | +4.5 | −8.3 |
| | 10 | −73.5 | −2.6 | −6.0 |
| "110" | 0.1 | −46.4 | −2.6 | −4.0 |
| | 1 | −48.8 | −2.2 | −3.1 |
| | 10 | −54.1 | −7.5 | −1.6 |
| BECLOME-TASONE DI-PROPIONATE | 0.1 | −0.6 | −1.4 | −3.3 |
| | 1 | −12.7 | +0.1 | −0.5 |
| | 10 | −55.0 | −1.7 | −4.7 |

Also within the scope of the invention are pharmaceutical compositions for use in the treatment of inflammatory conditions comprising an effective amount of one or more of the novel compounds of the invention, together with a compatible pharmaceutically-acceptable carrier.

The pharmaceutical dosage forms are prepared according to procedures well known in the art and, where suitable, may contain other active ingredients e.g. antibiotics.

The following, non-limiting, examples illustrate topical formulations prepared in accordance with the invention:

(a) *Inhalation aerosol*

| | |
|---|---|
| 6α-fluoroprednisolone-16α-methyl-17,21-diester | 1—10 mg |
| oleic acid | 0.5 mg |
| trichlorofluoromethane | 3000 mg |
| dichlorofluoromethane | 7500 mg |

(b) *Lotion*

| | |
|---|---|
| 6α-fluoroprednisolone-16α-methyl-17,21-diester | 0.05—5 mg |
| ethyl alcohol | 400 mg |
| polyethylene glycol 400 | 300 mg |
| hydroxypropyl cellulose | 5 mg |
| propylene glycol | 300 mg |

(c) *Glycol ointment*

| | |
|---|---|
| 6α-fluoroprednisolone-16α-methyl-17,21-diester | 0.05—5.0 mg |
| hexylene glycol | 100 mg |
| propylene glycol monostearate | 20 mg |
| white wax | 60 mg |
| white petrolatum | 880 mg |

The processes described above are illustrated in the Examples below but should not be construed as limiting the invention, equivalents thereof and products produced thereby which will be obvious to one skilled in the art being considered as part of the invention.

Preparation 1

To a suspension of 100 g of 9β,11β-epoxy-16β-methyl-pregna-1,4-diene-17,21-diol-3,20-dione, prepared as described in Journal of the American Chemical Society, *82*, 4012 (1960), in 800 ml of dry tetrahydrofuran and 150 ml of triethylorthopropionate at 20°C was added 1 g of para-toluenesulphonic acid. After 1 h the reaction mixture was neutralized with potassium acetate and diluted with water. The precipitate was collected, washed with water, and dried under vacuum to give in quantitative yield 9β,11β-epoxy-16β-methyl-pregna-1,4-diene-17,21-diol-3,20-dione 17,21-ethylorthopropionate. This latter compound was suspended in a mixture of 1 l of methanol, 0.5 l water and 5 ml glacial acetic acid and heated under reflux for 4 h. The reaction mixture was then cooled to 20°C and poured slowly into water (6 l) under strong agitation. The resulting precipitate was collected, washed with water, and dried under vacuum to give 9β,11β-epoxy-16β-methyl-pregna-1,4-diene-17,21-diol-3,20-dione 17-propionate (113 g), m.p. 150°C (d), $[\alpha]_D$ +47.5° (C = 1, $CHCl_3$). This compound has not previously been described.

In a similar manner, but instead of triethylorthopropionate using triethylorthoacetate, trimethylorthobutyrate, trimethylorthovalerate, or trimethylorthobenzoate, there were obtained the 17-acetate, 17-butyrate, 17-valerate, and 17-benzoate, respectively, of 9β,11β-epoxy-16β-methyl-pregna-1,4-diene-17,21-diol-3,20-dione.

In a similar manner, but starting from 9β,11β-epoxy-16α-methyl-pregna-1,4-diene-17,21-diol-3,20-dione, prepared as described in United States Specification No. 3,007,923, there were obtained the acetate, 17-propionate, 17-butyrate, 17-valerate, and 17-benzoate of 9β,11β-epoxy-16α-methyl-pregna-1,4-diene-17,21-diol-3,20-dione.

Preparation 2

To a solution of 113 g of 9β,11β-epoxy-16β-methyl-pregna-1,4-diene-17,21-diol-3,20-dione 17-propionate, prepared as described in Preparation 1, in 500 ml dry pyridine at 0°C was added dropwise 75 ml of propionic anhydride and the mixture was allowed to stand at 20—25°C for 3 h and then poured onto a mixture of ice/water/hydrochloric acid. The precipitate was collected, washed to neutrality with water, and dried under vacuum to give 122 g of 9β,11β-epoxy-16β-methyl-pregna-1,4-diene-17,21-diol-3,20-dione 17,21-dipropionate, m.p. 148°C, $[\alpha]_D$ +40° (C = 1, $CHCl_3$).

In a similar manner, but using the appropriate acyl or aroyl chloride or anhydride together with the appropriate 17-ester of 9β,11β-epoxy-16β-methyl-pregna-1,4-diene-17,21-diol-3,20-dione, prepared as described in Preparation 1, there were obtained the 17,21-diacetate, 17-butyrate 21-acetate, 17-valerate 21-acetate, and 17-benzonate 21-acetate of 9β,11β-epoxy-16β-methyl-pregna-1,4-diene-17,21-diol-3,20-dione.

In a similar manner, but starting from the appropriate 17-ester of 9β,11β-epoxy-16α-methyl-pregna-1,4-diene-17,21-diol-3,20-dione, prepared as described in Preparation 1, there were obtained the 17,21-diacetate, 17,21-dipropionate, 17-butyrate 21-acetate, 17-valerate 21-acetate, 17-benzoate 21-acetate of 9β,11β-epoxy-16α-methyl-pregna-1,4-diene-17,21-diol-3,20-dione.

Example 1

A mixture of 20 g of 9β,11β-epoxy-16β-methyl-pregna-1,4-diene-17,21-diol-3,20-dione 17,21-dipropionate, prepared as described in Preparation 2, and 2 g of paratoluensulphonic acid in 200 ml of isopropenyl acetate was heated under reflux for 2 h, neutralized with 20 g of potassium acetate, and evaporated to dryness under reduced pressure. The residue was dissolved in ethanol (200 ml) and treated at 0°C for 16 h with 10 g of perchloryl fluoride. The reaction mixture was then poured into 1 l of iced water and the resulting precipitate was collected, washed thoroughly with water, and dried under vacuum to constant weight (22 g) to give 6α-fluoro-9β,11β-epoxy-16β-methyl-pregna-1,4-diene-17,21-diol-3,20-dione 17,21-dipropionate. A sample crystallized from methanol had the following characteristics: m.p. 210°C $[\alpha]_D$ +47.5 (C = 1, $CHCl_3$)
$\nu_{max}$ 1765, 1740, 1675, 1640, 1620, 1250—1225 $cm^{-1}$
$\lambda_{max}$ 245 nm ($\varepsilon$ 16,500).

In a similar manner, but starting from the 17,21-diacetate 17-propionate 21-acetate, 17-butyrate 21-acetate, 17-valerate 21-acetate, and 17-benzoate 21-acetate diesters of 9β,11β-epoxy-16β-methyl-pregna-1,4-diene-17,21-diol-3,20-dione, prepared as described in Preparation 2, there were obtained the 17,21-diacetate, 17-propionate 21-acetate, 17-butyrate 21-acetate, 17-valerate 21-acetate, and 17-benzoate 21-acetate diesters, respectively, of 6α-fluoro-9β,11β-epoxy-16β-methyl-pregna-1,4-diene-17,21-diol-3,20-dione. (Table 2).

Example 2

Following the general procedure of Example 1 and making non-critical variations, but using 9β,11β-epoxy-16α-methyl-pregna-1,4-diene-17,21-diol-3,20-dione 17,21-diacetate, prepared as described in Preparation 2, as the reactant there was obtained 6α-fluoro-9β,11β-epoxy-16α-methyl-pregna-1,4-diene-17,21-diol-3,20-dione 17,21-diacetate in high yield. A sample crystallized from methanol had the following characteristics: m.p. 175—176°C
$\lambda_{max}$ 244 nm ($\varepsilon$ 16,800)
$[\alpha]_D$ +5° (C = 1, dioxane)

In a similar manner, but starting from the 17,21-dipropionate, 17-butyrate 21-acetate, 17-valerate 21-acetate, and 17 benzoate 21-acetate diesters of 9β,11β-epoxy-16α-methyl-pregna-1,4-diene-17,21-diol-3,20-dione, prepared as described in Preparation 2, there were obtained the 17,21-dipropionate, 17-butyrate 21-acetate, 17-valerate 21-acetate, and 17-benzoate 21-acetate diesters, respectively, of 6α-fluoro-9β,11β-epoxy-16α-methyl-pregna-1,4-diene-17,21-diol-3,20-dione (Table 2).

Example 3

To a solution of 6α-fluoro-9β,11β-epoxy-16β-methyl-pregna-1,4-diene-17,21-diol-3,20-dione 17,21-dipropionate (54.5 g), prepared as described in Example 1, in 500 ml glacial acetic acid at <5°C is added 18 ml of a 40% w/v solution of hydrogen bromide in acetic acid. The solution is kept 2 h at 0—5°C and then poured into water and the precipitate of 6α-fluoro-9α-bromo-16β-methyl-prednisolone 17,21-dipropionate is collected, washed with water and dried under vacuum at 30°C. The product, after trituration with 150 ml methyl isobutyl ketone had the following characteristics: m.p. 191—193°C $[\alpha]_D$ +85°.

A mixture of the above product (43.6 g), azobisisobutyronitrile (1.1 g), and tributyltin hydride (44 ml) in dry tetrahydrofuran (600 ml) was heated under reflux for 4 h and then evaporated to dryness.

The oily residue crystallized on treatment with heptane (250 ml) and the precipitate was collected, washed thoroughly with heptane, and then dried under vacuum to give 28.8 g of 6α-fluoro-16β-methylprednisolone 17,21-dipropionate. A sample crystallized from ethyl acetate had the following characteristics: m.p. 245—246°C, $[\alpha]_D$ +67.5° (C = 1, dioxan).

In a similar manner, but starting with the 17,21-diacetate, 17-butyrate 21-acetate, 17-valerate 21-acetate, and 17-benzoate 21-acetate diesters of 6α-fluoro-9β,11β-epoxy-16β-methyl-pregna-1,4-diene-17,21-diol-3,20-dione, prepared as described in Example 1, there were obtained the corresponding 17,21-diesters of 6α-fluoro-16β-methyl-prednisolone.

In a similar manner, but starting with the 17,21-diacetate, 17,21-dipropionate, 17-butyrate 21-acetate, 17-valerate 21-acetate, and 17-benzoate 21-acetate diesters of 6α-fluoro-9β,11β-epoxy-16β-methyl-pregna-1,4-diene-17,21-diol-3,20-dione, prepared as described in Example 2, there were obtained the corresponding 17,21-diesters of 6α-fluoro-16α-methyl-prednisolone (Table 3).

TABLE 2

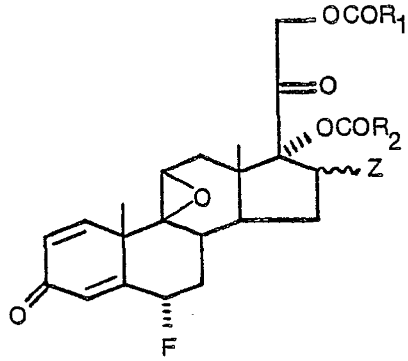

(II)

| $R_1$ | $R_2$ | Z | m.p.°C | $[\alpha]_D$ (solvent) |
|---|---|---|---|---|
| $C_2H_5$ | $C_2H_5$ | $\beta$-$CH_3$ | 210° | +47.5° (chloroform) |
| $CH_3$ | $CH_3$ | $\beta$-$CH_3$ | 229° (d) | +51° (chloroform) |
| $CH_3$ | $C_2H_5$ | $\beta$-$CH_3$ | 150° | +43° (chloroform) |
| $CH_3$ | $C_4H_9$ | $\beta$-$CH_3$ | 171° | +40° (chloroform) |
| $CH_3$ | $C_6H_5$ | $\beta$-$CH_3$ | 239—240° | +33° (chloroform) |
| $CH_3$ | $CH_3$ | $\alpha$-$CH_3$ | 175—176° | +5° (chloroform) |
| $C_2H_5$ | $C_2H_5$ | $\alpha$-$CH_3$ | 146—153° | +14° (chloroform) |

TABLE 3

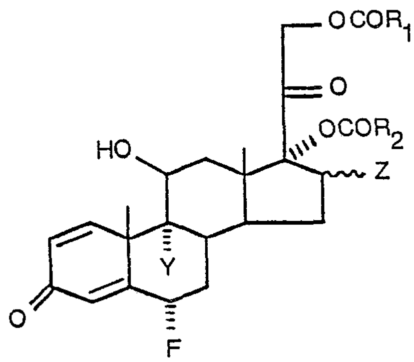

(I)

| $R_1$ | $R_2$ | Z | Y | m.p.°C | $[\alpha]_D$ |
|---|---|---|---|---|---|
| $C_2H_5$ | $C_2H_5$ | $\beta$-$CH_3$ | H | 245—246° | +67.5° |
| $C_2H_5$ | $C_2H_5$ | $\alpha$-$CH_3$ | H | 155—157° | +51° |
| $CH_3$ | $C_3H_7$ | $\alpha$-$CH_3$ | H | 241° | +54° |
| $CH_3$ | $C_4H_9$ | $\alpha$-$CH_3$ | H | 234° | +57° |

Rotation measured in dioxan
Melting points measured in open capillaries.

**Claims for the Contracting States: BE CH DE FR GB LI LU NL SE**

1. 6α-Fluoro-16-methyl-prednisolone 17,21-diesters of the formula I

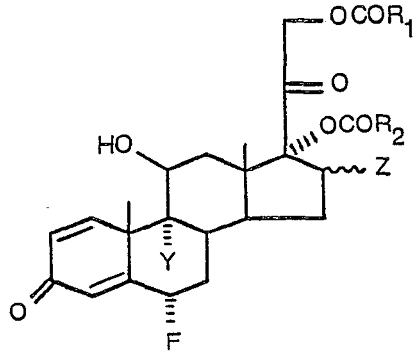

(I)

in which $R_1$ and $R_2$ are alkyl groups containing 1—6 carbon atoms or aryl groups, Y is a hydrogen, Z is a methyl group (either α- or β-oriented).

2. The compound, 6α-fluoro-16α-methyl-prednisolone 17,21-diacetate, as claimed in claim 1.

3. The compound, 6α-fluoro-16α-methyl-prednisolone 17,21-dipropionate, as claimed in claim 1.

4. The compound, 6α-fluoro-16α-methyl-prednisolone 17-valerate 21-acetate, as claimed in claim 1.

5. The compound, 6α-fluoro-16α-methyl-prednisolone 17-benzoate 21-acetate, as claimed in claim 1.

6. The compound, 6α-fluoro-methyl-prednisolone 17,21-diacetate, as claimed in claim 1.

7. The compound, 6α-fluoro-16β-methyl-prednisolone 17,21-dipropionate, as claimed in claim 1.

8. The compound, 6α-fluoro-16β-methyl-prednisolone 17-valerate 21-acetate, as claimed in claim 1.

9. The compound, 6α-fluoro-16β-methyl-prednisolone 17-benzoate 21-acetate, as claimed in claim 1.

10. Pharmaceutical compositions for use in the treatment of inflammatory conditions, comprising an effective amount of one or more compounds as claimed in claims 1—9, together with a comparable pharmaceutically-acceptable carrier or coating.

**Claims for the Contracting State: AT**

1. A method for the preparation of 6α-fluoro-prednisolone 17,21-diesters of the formula I

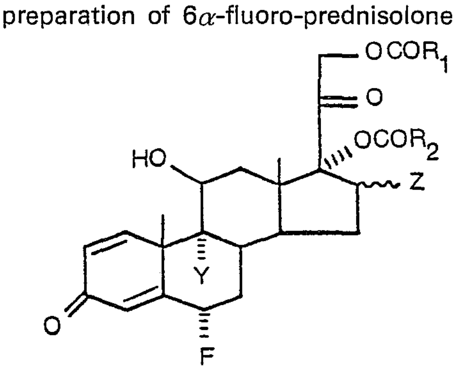

(I)

in which $R_1$ and $R_2$ are alkyl groups containing 1—6 carbon atoms or aryl groups, Y is hydrogen, Z is a methyl group (either α- or β-oriented), characterized in that compounds of the formula $I_I$

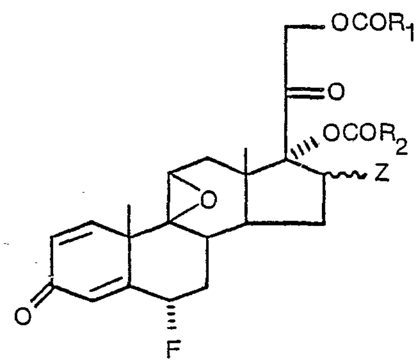

(II)

where $R_1$, $R_2$ and Z have the meanings given above, are hydrobrominated and, when Y = H, are subsequently reacted with tributyltin hydride.

2. A method as claimed in claim 1, characterized in that the hydrobromination is carried out with anhydrous HBr.

3. A method as claimed in claim 1, characterized in that the hydrobromination is carried out with acqueous HBr.

4. A method as claimed in claim 1, characterized in that the hydrobromination is carried out with a solution of HBr in glacial acetic acid.

5. A method as claimed in claim 1—4, characterized in that the hydrobromination is carried out at 0—5°C.

6. A method as claimed in claim 1, characterized in that the reaction with tributyltin hydride is carried out in the presence of an initiator of free radical reactions.

**Patentansprüche für die Vertragsstaaten: BE CH DE FR GB LI LU NL SE**

1. 6α-Fluor-16-methyl-prednisolon-17,21-diester der allgemeinen Formel I

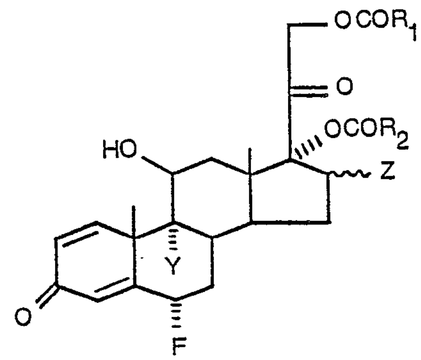

(I)

in der $R_1$ und $R_2$ Alkylreste mit 1—6 Kohlenstoffatomen oder Arylreste, Y ein Wasserstoffatom und Z eine Methylgruppe bedeutet (entweder α- oder β-orientiert).

2. 6α-Fluor-16α-methyl-prednisolon-17,21-diacetat, gemäß Anspruch 1.

3. 6α-Fluor-16α-methyl-prednisolon-17,21-dipropionat, gemäß Anspruch 1.

4. 6α-Fluor-16α-methyl-prednisolon-17-valerat-21-acetat, gemäß Anspruch 1.

5. 6α-Fluor-16α-methyl-prednisolon-17-benzoat-21-acetat, gemäß Anspruch 1.

6. 6α-Fluor-16β-methyl-prednisolon-17,21-diacetat, gemäß Anspruch 1.

7. 6α-Fluor-16β-methyl-prednisolon-17,21-dipropionat, gemäß Anspruch 1.

8. 6α-Fluor-16β-methyl-prednisolon-17-valerat-21-acetat, gemäß Anspruch 1.

9. 6α-Fluor-16β-methyl-prednisolon-17-benzoat-21-acetat, gemäß Anspruch 1.

10. Arzneimittel zur Verwendung bei der Behandlung von entzündlichen Zuständen, enthaltend eine wirksame Menge mindestens einer Verbindung gemäß Anspruch 1—9 zusammen mit einem verträglichen, pharmazeutisch annehmbaren Träger oder Beschichtung.

**Patentansprüche für den Vertragsstaat: AT**

1. Verfahren zur Herstellung neuer 6-fluor-16-methyl-prednisolon-17,21-diester der allgemeinen Formel

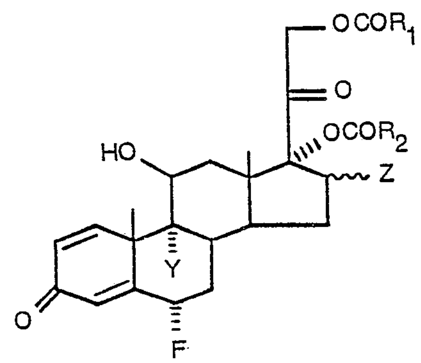

(I)

worin $R_1$ und $R_2$ Alkylgruppen mit 1—6 C-Atomen oder Arylgruppen, Y Wasserstoff und Z eine α-oder

β-orientierte Methylgruppe bedeuten, dadurch gekennzeichnet, daß Verbindungen der allgemeinen Formel

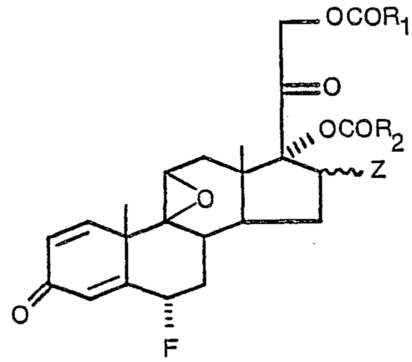

(II)

worin $R_1$, $R_2$ und Z obige Bedeutung haben, hydrobromiert, und, wenn Y für Wasserstoff steht, anschließend mit Tributylzinnhydrid umgesetzt werden.

2. Verfahren nach Anspruch 1, dadurch gekennzeichnet, daß die Hydrobromierung mit wasserfreiem HBr vorgenommen wird.

3. Verfahren nach Anspruch 1, dadurch gekennzeichnet, daß die Hydrobromierung mit wäßrigem HBr vorgenommen wird.

4. Verfahren nach Anspruch 1, dadurch gekennzeichnet, daß die Hydrobromierung mit einer Lösung von HBr in Eisessig vorgenommen wird.

5. Verfahren nach einem der Ansprüche 1—4, dadurch gekennzeichnet, daß die Hydrobromierung bei einer Temperatur von 0—5°C vorgenommen wird.

6. Verfahren nach Anspruch 1, dadurch gekennzeichnet, daß die Umsetzung mit Tributylzinnhydrid in Gegenwart eines Initiators freier Radikalreaktionen vorgenommen wird.

**Revendications pour les Etats contractants: BE CH DE FR GB LI LU NL SE**

1. 6α-fluoro-16-méthyl-prednisolone 17,21-diesters de formule I

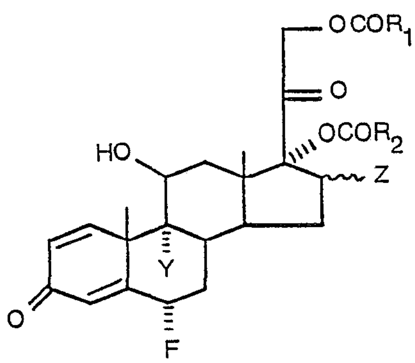

(I)

dans laquelle $R_1$ et $R_2$ représentent des groupes alkyle contenant 1 à 6 atomes de carbone ou des groupes aryle, Y est un hydrogène, Z est un groupe méthyle (orienté soit en α soit en β).

2. Le composé 6α-fluoro-16α-méthyl-prednisolone 17,21-diacétate, tel que revendiqué dans la revendication 1.

3. Le composé 6α-fluoro-16α-méthyl-prednisolone 17,21-dipropionate, tel que revendiqué dans la revendication 1.

4. Le composé 6α-fluoro-16α-méthyl-prednisolone 17-valérate 21-acétate, tel que revendiqué dans la revendication 1.

5. Le composé 6α-fluoro-16α-méthyl-prednisolone 17-benzoate 21-acétate, tel que revendiqué dans la revendication 1.

6. Le composé 6α-fluoro-16β-méthyl-prednisolone 17,21-diacétate, tel que revendiqué dans la revendication 1.

7. Le composé 6α-fluoro-16β-méthyl-prednisolone 17,21-dipropionate, tel que revendiqué dans la revendication 1.

8. Le composé 6α-fluoro-16β-méthyl-prednisolone 17-valérate 21-acetate, tel que revendiqué dans la revendication 1.

9. Le composé 6α-fluoro-16β-méthyl-prednisolone 17-benzoate 21-acétate tel que revendiqué dans la revendication 1.

10. Compositions pharmaceutiques pour utilisation dans le traitement des états inflammatoires,

comprenant une quantité efficace d'un ou plusieurs composés tels que revendiqués dans les revendications 1—9, avec un véhicule ou revêtement compatible et pharmaceutiquement acceptable.

**Revendications pour l'Etat contractant:AT**

1. Procédé de préparation de 6$\alpha$-fluoro-prednisolone 17,21-diesters de formule I

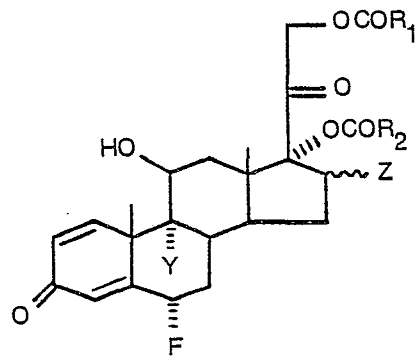

(I)

dans laquelle $R_1$ et $R_2$ représentent des groupes alkyle contenant 1 à 6 atomes de carbone ou des groupes aryle, Y est un hydrogène, Z est un groupe méthyle (orienté soit en $\alpha$ soit en $\beta$), caractérisé en ce que des composés de formule II

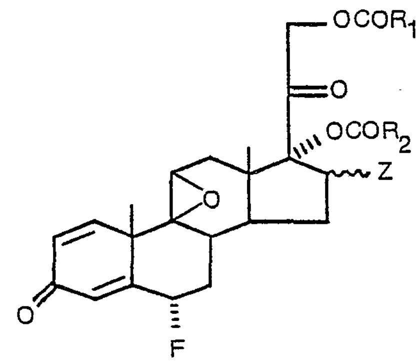

(II)

dans laquelle $R_1$, $R_2$ et Z ont les mêmes significations que ci-dessus, sont soumis à une bromhydratation et, dans le cas où Y = H, sont ensuite amenés en réaction avec l'hydrure de tributylétain.

2. Procédé selon la revendication 1, caractérisé en ce que la bromhydratation est réalisée à l'aide de HBr anhydre.

3. Procédé selon la revendication 1, caractérisé en ce que la bromhydratation est réalisée à l'aide de HBr aqueux.

4. Procédé selon la revendication 1, caractérisé en ce que la bromhydratation est réalisée à l'aide d'une solution solution de HBr dans l'acide acétique glacial.

5. Procédé selon les revendications 1 à 4, caractérisé en ce que la bromhydratation est effectuée à 0—5°C.

6. Procédé selon la revendication 1, caractérisé en ce que la réaction avec l'hydrure de tributylétain est réalisée en présence d'un initiateur de réactions par radicaux libres.